Europäisches Patentamt

(19) European Patent Office

Office européen des brevets

(11) **EP 1 124 602 B1**

(12) **EUROPEAN PATENT SPECIFICATION**

(45) Date of publication and mention
of the grant of the patent:
**20.04.2005 Bulletin 2005/16**

(51) Int Cl.⁷: **A61M 11/06**, A61M 15/00,
A61B 5/087

(21) Application number: **99950992.0**

(22) Date of filing: **26.10.1999**

(86) International application number:
**PCT/GB1999/003540**

(87) International publication number:
**WO 2000/024445 (04.05.2000 Gazette 2000/18)**

(54) **DRUG DELIVERY APPARATUS**

VORRICHTUNG ZUR ABGABE VON MEDIKAMENTEN

APPAREIL D'ADMINISTRATION DE MEDICAMENT

(84) Designated Contracting States:
**AT BE CH CY DE DK ES FI FR GB GR IE IT LI LU
MC NL PT SE**
Designated Extension States:
**AL LT LV MK RO SI**

(30) Priority: **26.10.1998 GB 9823434**

(43) Date of publication of application:
**22.08.2001 Bulletin 2001/34**

(60) Divisional application:
**05075006.6**

(73) Proprietor: **Profile Respiratory Systems Limited
Bognor Regis, West Sussex PO22 9SL (GB)**

(72) Inventors:
• **DENYER, Jonathan, Stanley, Harold
Chichester, West Sussex PO19 3PW (GB)**
• **DYCHE, Anthony
Hayling Island, Hampshire PO11 0ND (GB)**
• **PRINCE, Ivan, Richard
Hedge End, Southampton SO30 0FD (GB)**

(74) Representative:
**Wright, Howard Hugh Burnby et al
Withers & Rogers,
Goldings House,
2 Hays Lane
London SE1 2HW (GB)**

(56) References cited:
EP-A- 0 667 168          WO-A-92/11054
WO-A-97/48431            DE-A- 3 636 669
GB-A- 2 077 444          GB-A- 2 294 402
US-A- 3 741 208

**Description**

This invention relates to drug delivery apparatus, particularly, but not exclusively, nebulisers and dosimetric spacers.

Many different types of nebulisers are known for delivering medication directly into the lungs of a patient, usually for treatment of respiratory diseases. Nebulisers normally deliver medication in the form of droplets or a dry powder. In most nebulisers, atomisation of the medicament into a stream of air occurs continuously, regardless of whether the patient is inspiring or expiring. However, the effect of continuous atomisation is that a significant proportion of the medication is lost during expiration.

Commonly known nebulisers are either pneumatically operated from a compressed air source connected to the nebuliser which atomises the liquid, or are ultrasonic nebulisers which use a piezo-electric crystal to atomise the liquid. More recently, a mesh-type nebuliser has been developed in which the medication is forced through a fine mesh in order to create droplets of the medication. A further type of nebuliser, or inhaler, is one which uses a piezo-electric vibrator together with an electro-static charge plate to fluidise and disperse a dry powder aerosol into an airstream. Such a nebuliser is disclosed in US 5694920.

The optimum diameter of medication particles or droplets is about 1-5 microns. If the particles or droplets are bigger than this, they are likely to be impacted in the airway before they reach the lungs, but if they are smaller than one micron, they tend to be carried out of the lungs again on exhalation without sedimenting in the lungs.

Nebulisers and inhalers disperse the small particles of medication into an air stream, or stream of other gas, leading to a patient. References to the air which carries the medication entrained in it includes other gases suitable for carrying the medication.

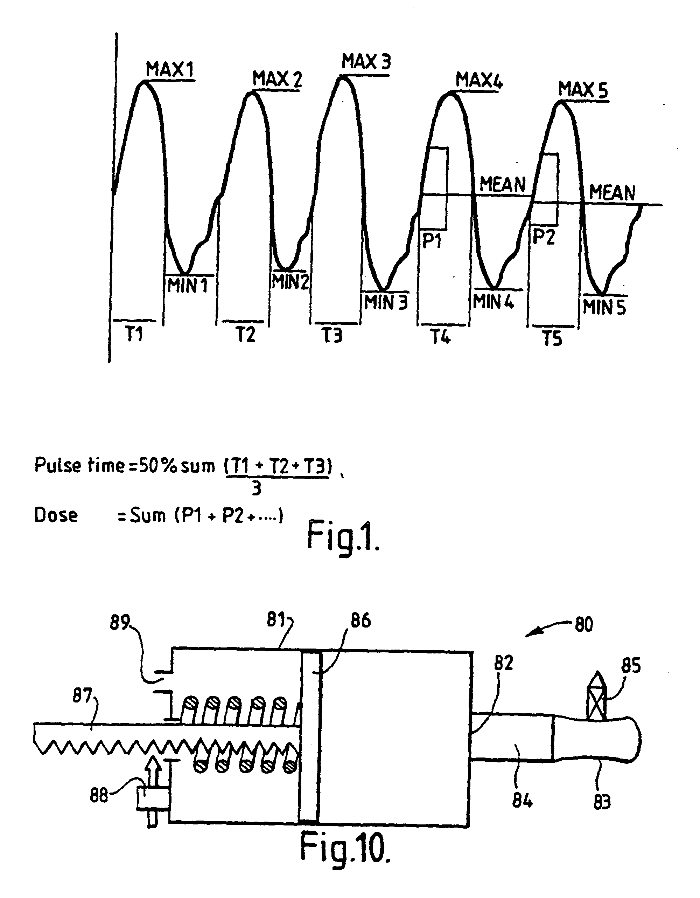

One known nebuliser analyses the pressure changes within the device during the first three breaths to determine an average shape of the breathing pattern. A timed pulse of atomisation is commenced when starting subsequent inspirations such that atomisation occurs for the first 50% of the inspiration. This is illustrated in Figure 1 where the breathing pattern and pulse are superimposed. This is effective in reducing the loss of medication during exhalation to about 3%. Figure 1 shows the breaths in a graph of flow rate against time. When the treatment is commenced, a patient breathes in and out three times through the nebuliser before treatment commences. The first three breaths are measured so that the timed pulse of atomisation occurs for 50% of the average time of inhalation. The duration of inhalation is indicated as T1, T2 and T3. These timed periods are averaged, and divided by two in order to determine the pulse length for the next fourth breath where treatment starts. For each subsequent breath, the duration of the pulse of atomisation is determined by summing the time period of inhalation of the previous three breaths, dividing by three to obtain an average and dividing by two. The dose administered to the patient is directly proportional to the duration of the pulse of atomisation, and so the period of atomisation is summed, and the atomiser is switched off, or indicates that the patient should stop once the dose administered to the patient reaches the amount of medication prescribed for that treatment.

Other nebulisers are known in which the timed pulse of atomisation is fixed to be other than 50% of the duration of inspiration. However, in these other nebulisers, the pulse length must be set for each patient by the clinician. Many of the nebulisers are, therefore, suitable only for use in a controlled environment, such as a hospital. The setting of the pulse length for each patient means that most nebulisers are not suitable for a patient to use at home.

DE 3636669 discloses an apparatus for delivering aerosol to the airways of a patient which includes separate inspiration and expiration lines. An aerosol generator is located in the inspiration line which generates an aerosol only during expiration of the patient so that the aerosol fills a region in the inspiration line. On inspiration by the patient, the aerosol from the inspiration line is drawn into the lungs of the patient However, the volume of the aerosol is fixed . The volume of aerosol is fixed in order to be appropriate for the assumed tidal volume when a patient breathes in a specific manner.

GB 2077444 discloses an apparatus suitable for use in determining at least two parameters of a human or animal respiratory system. The apparatus includes a pressure sensor, a flow sensor, a volume sensor, a monitoring unit and a calculating unit whereby during each respiratory cycle, the monitoring unit supplies the calculating unit with at least two sets of sensed values of the pressure the flow rate and the volume and the calculating unit calculates the required parameters of the respiratory system from the sets of sensed values. The volume is calculated conventionally by integrating the rate of flow over time.

Reference is made to our co-pending International Patent Publication No. WO 97/48431. Figures 2 and 3 of this application show the nebuliser which is disclosed in the above co-pending Patent application. Referring to Figure 2, a mouthpiece 1 is shown through which a patient inhales in the direction of arrow 2. Below the mouthpiece 1 is removable atomising section 3 which, in turn, rests on a base 4.

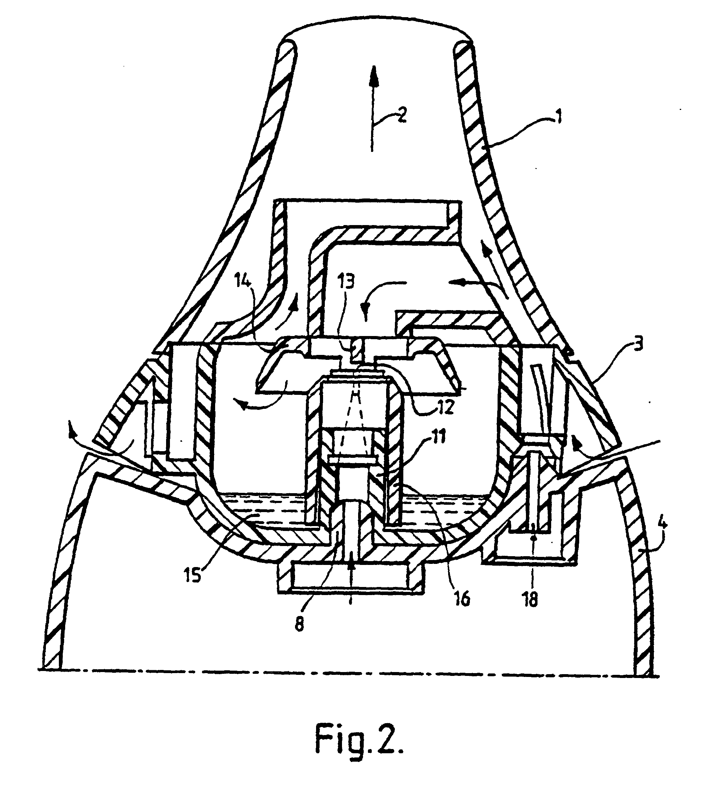

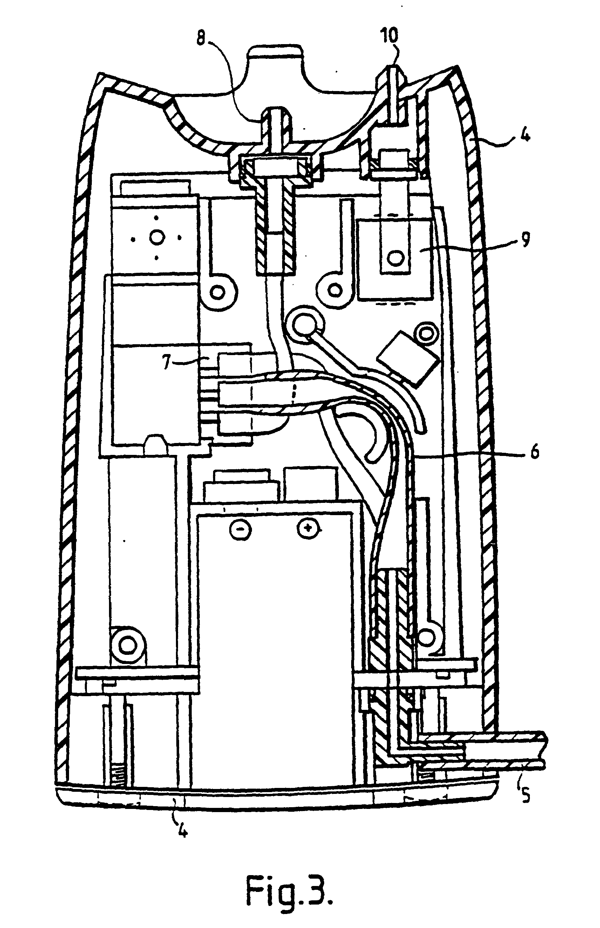

The base 4 is shown in more detail in Figure 3. Referring to Figure 3, the base 4 includes an inlet 5 through which air is supplied under pressure from a compressor (not shown). The pressurized air is led via a tube 6 to a manifold 7 which controls the flow of pressurized air to an air outlet 8 which directs air into the atomising section 3 shown in Figure 2. The base 4 also includes a pressure sensor 9 which detects the pressure within the atomising section 3 via a port 10.

Referring again to Figure 2, air under pressure passes through the air outlet 8 of the base 4 and is conducted through a tubular post 11 to an atomiser nozzle 12 out of which the air issues under pressure. A deflector 13 is located in the path of the pressurised air issuing from the nozzle 12 so that the pressurized air is deflected laterally so as to pass beneath a baffle 14. The passage of the pressurized air across the top of the tubular post 11 causes medication 15 to be drawn up between the outer surface of the tubular post 11 and the inner surface of a sleeve 16 which surrounds the tubular post 11. The medication 15 is atomised in the stream of air, and carried away in the stream of air below the rim of the baffle 14 and up through the mouthpiece 1 to a patient.

The pressure sensor 9 in the base 4 monitors the breathing pattern of a patient, and on the basis of the breathing pattern, the manifold 7 is controlled to supply pressurized air to the atomising section 3 only during the first 50% of an inhalation phase.

Whilst a particular type of nebuliser is described above, the present application is suitable for application to any type of nebuliser.

The invention also relates to other drug delivery apparatus, such as spacers in which a dose of a drug in droplet or powder form is released into a spacer chamber or holding chamber from which the patient inhales. These are most appropriate for elderly patients or children who have difficulty in using a multi-dose inhaler or dry powder inhaler, for example, because they have trouble coordinating the release of the drug with the beginning of inhalation, or because their inhalation flow rates are too small. For example, spacers are disclosed in International patent publication number WO 96/13294.

According to a first aspect of the present invention, a drug delivery apparatus comprises delivery means for delivering medication-laden air and air not carrying any medication to a patient for inspiration; monitoring means for monitoring a patient's breathing pattern; and control means for controlling the said delivery means to selectively deliver the medication-laden air and air not carrying any medication, characterised in that the control means is arranged to control the delivery means to deliver the medication-laden air in pulses, the length of which, and their proportion of the inspiratory phase of the breathing pattern being varied by the control means depending on the breathing pattern monitored by the monitoring means.

A method for determining the duration of a pulse during which medication-laden air is delivered to a patient during inspiration comprises:

(i) measurement of the tidal volume of a patient;
(ii) measuring the duration of inspiration of a patient;
(iii) storing an estimate of the volume of a patient's upper airway; and
(iv) calculating the duration of the pulse on the basis of the measured tidal volume of the patient, the measured duration of inspiration and the stored estimated volume of the patient's upper airway.

In this document, the upper airways of a patient are the mouth and trachea, and where a nebuliser is used, preferably include the volume of the nebuliser chamber.

The determination of the length of pulse enables the proportion of the inhalation time during which atomisation occurs to be extended above 50% towards 100%. This will result in the patient receiving their treatment in a shorter time, since it will take fewer breaths to deliver the required dose of medication. However, there is no point in continuing delivering the medication into air which is inhaled by the patient at the end of his or her inspiratory phase (the 'end volume'), since it will remain in the upper airways. The medication which does not go beyond the upper airways will be wasted when the patient exhales.

Thus, the invention enables a pulse of to be generated which is longer than 50% but which stops before the end volume of inspiration begins. Another advantage of this invention is that a patient's adherence to the treatment regime will be much improved if the length of treatment is reduced.

In addition, the invention allows automatic optimisation of the pulse length without it needing to be set by a clinician. This means that the pulse length will automatically be adapted to each patient on the basis of the patient's breathing pattern at the time the medication is being administered. Thus, a nebuliser or other drug delivery apparatus may be used by the patient outside of the controlled environment of a hospital, and may be used at home. In addition, it is possible for the apparatus to indicate when a dose has been administered without the patient needing to count the number of breaths which he or she has taken.

According to the preferred embodiment, the means for measuring the tidal volume of a patient includes means for measuring a patient's peak flow, and tidal volume prediction means for calculating the tidal volume on the basis of the peak flow from the peak flow measuring means, and the duration of inspiration measured by the timing means.

Some or all of the values used in the calculations are mean values derived from a number of earlier measurements of each breathing pattern of the patient. For example, the patient will start inspiration through the apparatus, and the medication will not be delivered during the first three breaths. The first three breaths are analysed by recording the duration of inspiration, and the peak flows during inhalation as are required to determine the duration of a pulse of atomisation. Delivery of the medication takes place on the fourth and subsequent breaths, in each case the values in the calculations are derived from a number of earlier measurements of the inspiration phase of a patient, in this case the previous three inspiratory phases.

Preferably, where the apparatus is a nebuliser, the atomisation is caused by a stream of gas under pressure passing through the nebuliser and sourced from a gas supply means. This gas is normally air, and the source is preferably a compressor operating together with an accumulator. During atomisation, gas from the accumulator is used to atomise the medication, and the compressor generates air under pressure to fill the accumulator. If a patient's inspiration is very long, the accumulator may be caused to be emptied, thereby disrupting atomisation. The atomiser, therefore, preferably includes a means for limiting the duration of the pulse so as to maintain the accumulator in a state where it is always under some pressure. In addition, the accumulator may include a valve which, when the accumulator is full, vents gas to atmosphere thereby preventing it from becoming dangerously full. It is often preferable to maintain the compressor in operation all the time and to vent excess air to atmosphere rather than to switch the compressor on and off.

A method of predicting the tidal volume of a patient comprises:

(i) measuring a patient's peak flow;
(ii) measuring the duration of inspiration of the patient;
(iii) calculating the tidal volume on the basis of the measured peak flow, and the measured duration of inspiration of the patient.

Measuring the patient's respiratory volume (tidal volume) has previously involved continually monitoring the patient's inspiratory flow, typically every ten milliseconds. The flow rate is integrated over the duration of inspiration to determine the inspiratory volume. However, according to the invention, the tidal volume of a patient can be determined much more simply. This invention reduces the amount of data processing which is required, thereby reducing the cost of the overall nebuliser. The peak flow is much simpler to measure, and can be used more simply in a calculation to determine the tidal volume.

Embodiments of the present invention are described below by way of example, and with reference to the drawings in which:

Figure 1 is a graph showing the inhalation pattern of a patient over time, and indicating when the pulse of atomisation occurs in the first 50% of inspiration, as occurs in one known nebuliser;

Figures 2 and 3 show a known nebuliser which generates pulses of atomisation during the first 50% of inspiration;

Figure 4 is a flow diagram showing how the pulse of atomisation during inspiration is determined;

Figure 5 is a graph showing the predicted tidal volume against measured tidal volume;

Figure 6 is a flow diagram showing the limitation of the pulse length depending on the supply of pressurised gas;

Figure 7 shows the nebuliser together with a source of pressurised gas;

Figure 8 shows an air accumulator within the air supply;

Figure 9 is a schematic drawing showing the way in which the nebuliser is controlled; and

Figure 10 is a schematic drawing of a dosimetric spacer according to the present invention.

This invention applies, amongst other things, to nebulisers of the type which generate pulses of atomisation, as in the prior art nebuliser described above. The invention is not, however, limited to the exact nebuliser described above, but may be applied to other nebulisers. For convenience, the description below of the present invention will refer to components of the prior art device shown in Figures 2 and 3, and because many of the components, for example, the manifold, may be used in the present invention. The nebuliser may be one of a jet nebuliser, ultrasonic nebuliser or a pressure mesh nebuliser.

Jet nebulisers are of two kinds, these being air-jet nebulisers and liquid-jet nebulisers. An example of an air-jet nebuliser, which uses a source of compressed air to nebulise a liquid, is disclosed in EP 0627266 (Medic-Aid Limited).

An example of a liquid-jet nebuliser, which drives a liquid through one or more nozzle outlets to produce a spray of fine droplets is disclosed in WO 94/07607 (Boehringer Ingelheim International GmbH et al).

Ultrasonic nebulisers which nebulise a liquid using ultrasonic waves usually developed with an oscillating piezo-electric element, take many forms, these including nebulisers where liquid is in direct contact with the piezo-electric element, where there is an amplifying interface, typically an enclosed fluid, between the piezo-electric element and the liquid, and where the piezo-electric element vibrates a mesh from which an aerosol is generated. Examples of ultrasonic nebulisers are disclosed in US 4533082 (Maehara et al) and US 5261601 (Ross et al).

The nebulisers described in those documents include a housing that has a reservoir which holds a quantity of liquid to be dispensed, which housing has a perforated membrane in contact with the reservoir and an ultrasonic vibrator connected to the housing to vibrate the perforated membrane. Another example of an ultrasonic nebuliser is described in WO 97/29851 (Fluid Propulsion Technologies, Inc). An example of a pressure mesh nebuliser, which may or may not include a piezo-electric element, is disclosed in WO 96/13292 (Aradigm Corporation).

Extending the proportion of the inhalation of the patient in which atomisation takes place above 50% results in the patient receiving their treatment faster since it will take fewer breaths to deliver the required volume of medication. However, to avoid wastage of the medication which is atomised in the end volume of patient's inspiratory volume, the pulse of atomisation must be stopped before the end volume is reached. The end volume is the volume of air inhaled by a patient at the end of the inspiratory volume which remains in the upper airways (the mouth and trachea) and does not enter into the lower parts of the lungs. Medication which is atomised into the end volume is wasted when the patient exhales, together with any air atomised medication left in the nebuliser, since it does not reach the lungs.

The end volume is the volume of the patient's upper airway, and is proportional to the size of the patient. Clearly, the end volume will vary as a percentage of the inspiratory tidal volume since the tidal volume changes significantly depending on the type and extent of the respiratory disease suffered by the patient. The optimum duration of atomisation pulse would, therefore, be from the start of inhalation up to the point during inspiration when the volume remaining to be inspired equals the end volume. Atomisation would then be stopped and the remaining end volume would clear the atomised medication from the device and the upper airways of the patient and into the lungs. Thus, the percentage of inspiration in which atomised medication is delivered is maximised, thereby minimising treatment time and still avoiding wastage of medication. The length of the atomisation pulse is dependent upon the patient's inspiratory tidal volume. The nebuliser must therefore measure the patient's tidal volume, preferably on a breath by breath basis so as to calculate, for example from the previous three breaths, an average inhalation volume for the next breath. Thus, the atomisation pulse time will be calculated as follows:

$$\text{Pulse time} = \text{mean inspiratory time} \times \frac{(\text{mean tidal volume - end volume})}{\text{mean tidal volume}}$$

Timing means are included in the nebuliser connected to the pressure sensor 9 (shown in Figure 3) in order to measure the duration of inspiration. Storage means are also included in the nebuliser in which an estimate of the end volume of a particular patient is stored. Since this figure is a constant value for a particular patient, this can be entered at the beginning of a course of treatment, and is estimated on the basis of the size of the patient. The nebuliser includes a means for measuring the tidal volume of a patient. According to one form of the invention, the patient's inspiratory flow is monitored continuously, typically every ten milliseconds, and this is integrated over the inspiratory duration. Another, simpler, way of measuring the tidal volume of a patient is described later in this specification.

The nebuliser also includes means for calculating the atomisation pulse time on the basis of the duration of inspiration, the tidal volume and the end volume. The calculation means carries out the calculation outlined above.

In view of the fact that the nebuliser adapts to the breathing pattern of a patient, when the patient starts breathing, no atomisation takes place during the first three breaths. Those first three breaths are used to analyse the breathing pattern of the patient. The flow rate of the first three breaths are measured, and from this, the duration of the inhalation phase of the first three breaths are calculated, and an average found. The average duration of inhalation is then used in the calculation to determine the pulse length of atomisation during the fourth breath. In addition, as the patient continues to breathe in and out, the previous three breathing patterns are measured and used to calculate the next pulse duration. Thus, if a patient's breathing pattern improves during treatment, the nebuliser will adapt to this change in order to optimise the dose administered during each breath.

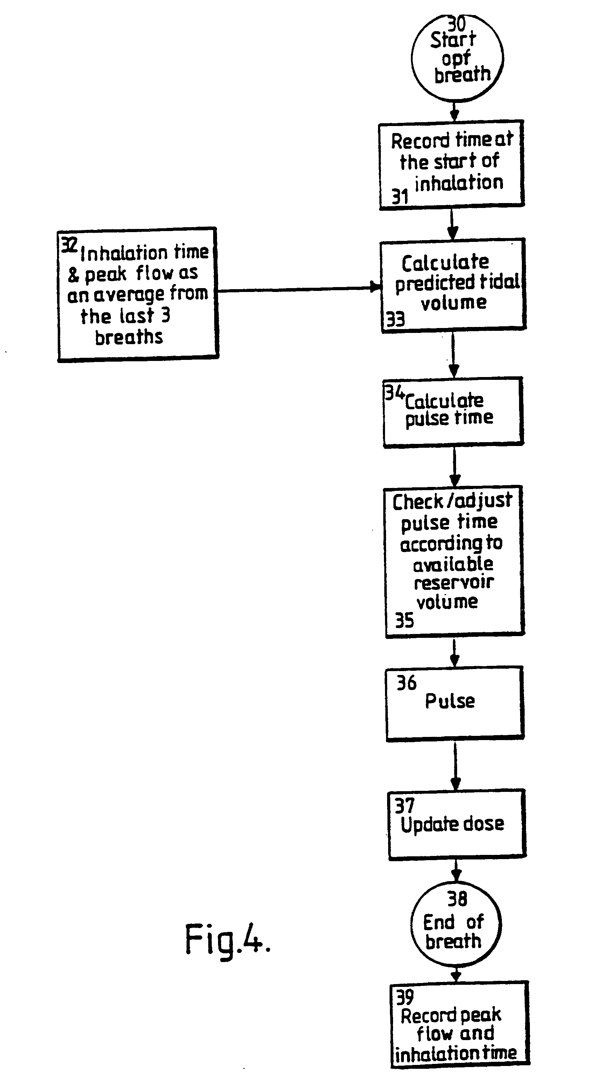

Referring now to Figure 4, the steps taken by the nebuliser, and by the patient are described. The first operation, box 30 represents the patient starting to inhale. The timing means records the time at which inhalation starts as shown in box 31, and during inhalation, a calculation is performed to predict the tidal volume of the patient as shown in box 33. This step will be described in more detail later in a specification, but it will be noted that the calculation requires data to be included in the calculation which is the inhalation time and peak flow as an average from the last three breaths, as shown in box 32. The pulse time is then calculated by the calculation means as shown in box 34, and the pulse time is adjusted, as shown in box 35 in the event that the pulse length would exhaust an accumulator from which is pressurised air is supplied to the nebuliser. This step, shown in box 35 is also described in more detail later in this specification. The pulse of atomisation occurs during inhalation, and after it has stopped, a calculation is carried out to determine what dose has been atomised. At the end of the breath as shown in box 38, details of the peak flow of the patient inhalation, and the duration of inhalation are recorded so that calculations determining pulse length may be made for subsequent breaths. This is shown in box 39.

Reference is made above to the simpler prediction of tidal volume. As will be appreciated, measuring tidal volume by integrating measured flow rate over the time of inspiration requires considerable processing power and is relatively expensive. A simpler method of determining the tidal volume is proposed which requires much simpler calculations and much simpler measurements to be made for use in such a calculation. To carry out the measurement, the nebuliser includes a peak flow detector for detecting the peak flow rate of inspiration.

The calculated, or predicted tidal volume is derived from the peak flow measured by the peak flow detector, and the duration of inspiration measured by the timer. The tidal volume calculation means carries out the following calculation:

$$\text{Predicted tidal volume} = C \times \text{Mean Peak Flow} \times \frac{\text{Inspiratory Time}}{60}$$

C is a constant and it is found that C = 0.7

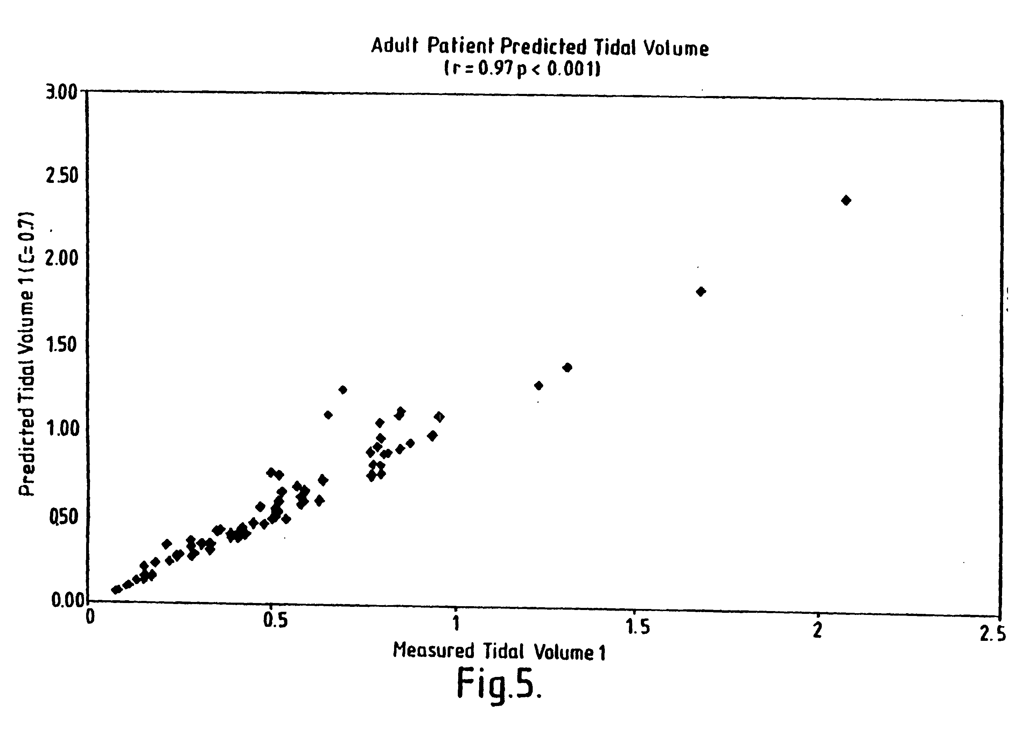

Figure 5 is a graph of the predicted tidal volume against measured tidal volume . Each point on the graph represents a patient whose tidal volume has been measured by a complex tidal volume calculation by integration of the patient's inspiratory flow over the duration of inhalation; and the predicted tidal volume according to the new, simpler method of calculation. It will be seen that the predicted tidal volumes are extremely accurate, and so the predicted tidal volume may be included in the calculation of atomisation pulse time.

The use of a low flow rate compressor together with an accumulator to supply compressed air to the nebuliser is disclosed in our earlier Patent application published as WO 97/48431, which is referred to above. In the past, the size of the compressor and accumulator are selected so that the maximum pulse that can be delivered by the device (currently 50% of inspiratory time) does not exceed the accumulator volume for any given pulse or the mean output of the compressor. Now that the pulse time is variable, it is preferable to calculate the maximum pulse time available from the air supply system. For patients who have a slightly higher inspiratory demand, the pulse time of atomisation will be reduced so that the supply capability of the air supply system is not exceeded. The calculations are carried out on a breath by breath basis, assuming that the accumulator is filled at constant flow rate from the compressor. The volume of air added to the accumulator from the end of previous pulse to the start of the next pulse is calculated and then added to the volume remaining at the end of the previous pulse.

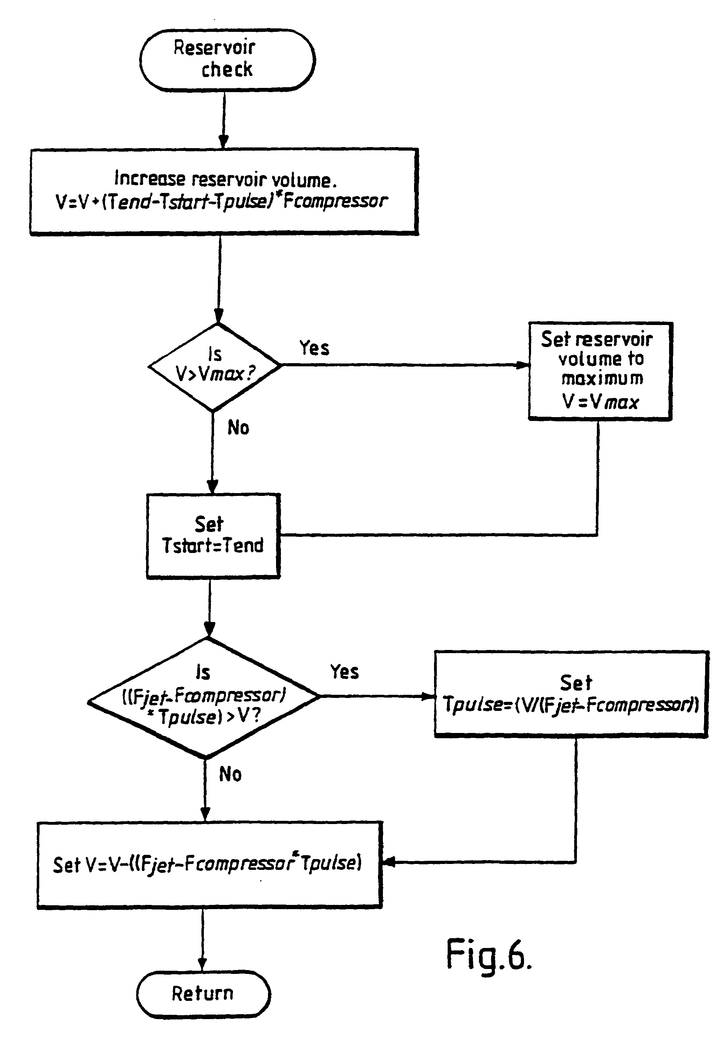

Figure 6 is a flow diagram showing the calculations carried out in ensuring that the volume of air used does not exceed the volume of the accumulator. If the air in the accumulator is calculated to be above the maximum volume of the accumulator, the volume is set to be at its maximum $V = V_{max}$. This is because there is an automatic vent valve which limits the volume of air stored in the accumulator. The maximum pulse time can then be calculated on the basis of the rate of flow of air out of the accumulator which is the flow to the atomiser jet, minus the flow rate to the compressor. If this exceeds the volume available in the accumulator, then the pulse time is limited to the current accumulator volume. The volume of accumulator at the end of the pulse is then calculated to be used at the beginning of the next calculation occurring at the beginning of the next inhalation of the patient. Thus, the maximum pulse time for individual breaths is calculated without exceeding the capacity of the air supply system. The compressor has a constant output flow rate, typically 1.5 litres per minute and the nebuliser jet has a flow rate of 6 litres per minute during pulsing. The accumulator has a volume of approximately 150 millilitres at NTP.

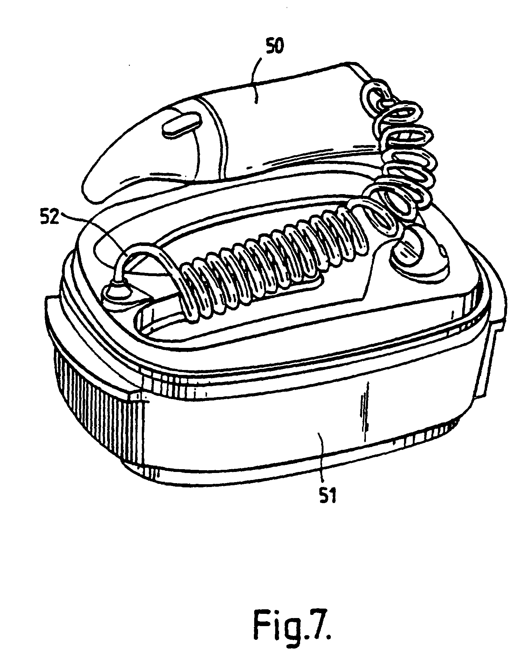

Figure 7 shows the nebuliser 50 connected to the air supply 51 by a flexible tube 52.

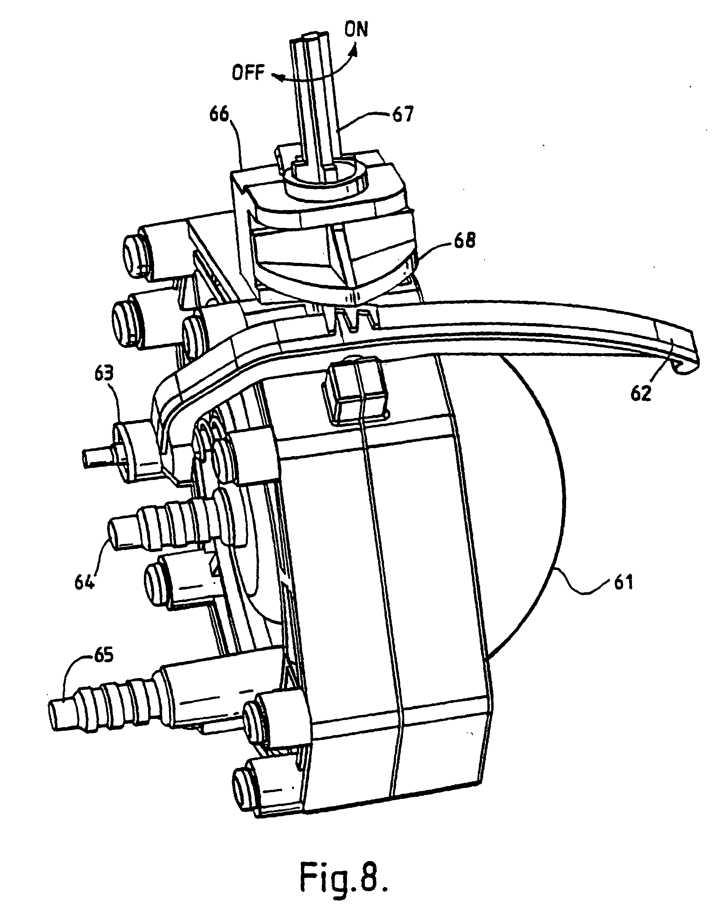

Referring to Figure 8, the accumulator is shown which has a vent 63, thereby limiting the maximum expansion of the accumulator. As each pulse is delivered to the nebuliser, the diameter of the accumulator is reduced, and the vent 63 is closed.

The compressor may be mains powered or battery powered. The pump, especially a mains powered pump, operates continually during use, and operates to inflate the accumulator. When the pressure in the accumulator reaches the required level, a pressure switch in the hand-held part of the nebuliser is activated as is described in an earlier Patent application referred above. That switches the nebuliser ON. Once the treatment has been completed, the compressor is switched OFF. The accumulator deflates and the pressure switch in the hand-held part of the nebuliser deactivates the unit.

Referring to Figure 8, the pump supplies air to the accumulator via a port 64. Inflation of the diaphragm 61 of the accumulator is controlled by an assembly including an arm 62 which is connected to a vent valve 63. When the diaphragm 61 of the accumulator reaches the maximum desired extension, it contacts the arm 62 to open the vent valve 63. This releases to atmosphere the flow of air from the compressor, and maintains the accumulator at a fixed extension. During use, air is removed from the accumulator via port 65 and the diaphragm 61 shrinks and loses contact with the vent arm 62 which closes the valve 63 allowing the compressor to recharge the accumulator until the vent arm 62 again operates the vent valve 63.

It is also advantageous to vent the accumulator to atmosphere when the compressor is switched off, and this is achieved by mounting the main power switch 66 on top of the accumulator with a rotary knob 67. The bottom of the knob 67 includes a cam 68 which contacts the vent arm 62 to open the vent 63 thereby releasing pressure from the accumulator. Simultaneously, the compressor is switched off. When the compressor is switched back on again, the cam 68 is disengaged from the vent arm 62, thereby closing the vent valve 63.

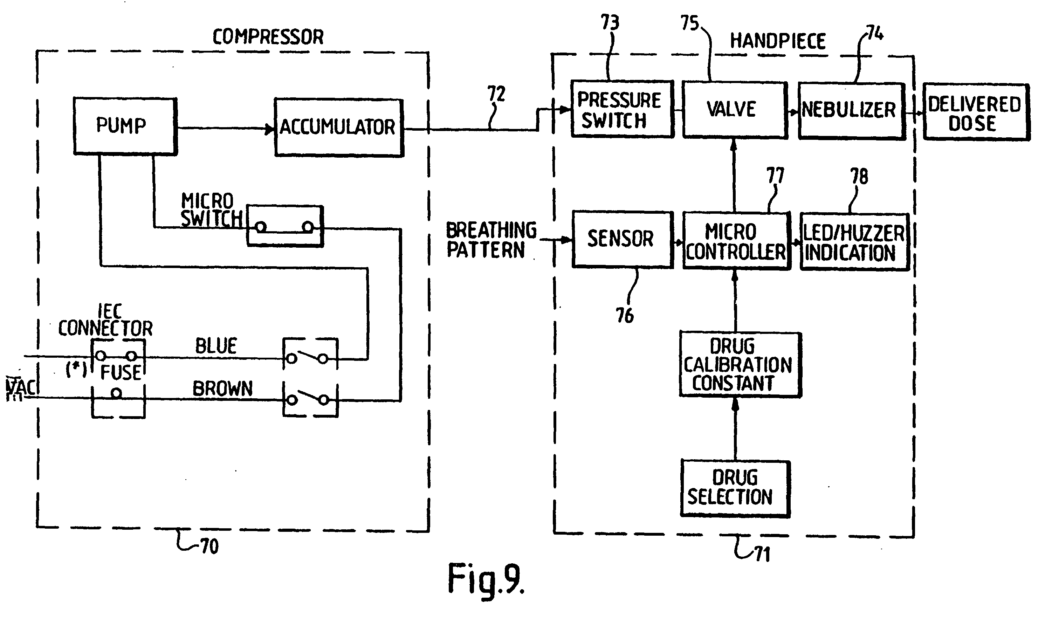

Figure 9 illustrates a simplified form of the way in which all of the components of the nebuliser are connected together. The compressor and accumulator 70 are shown as being separate from the hand-held part of the nebuliser 71, but connected by a tube 72 carrying the pressurised air into the nebuliser 71. In the compressor and accumulator part 70, the pump is shown to supply the accumulator 70 with compressed air. In the nebuliser part 71, the nebuliser is switched on at the pressure switch 73 by the presence of pressurised air in tube 72. The nebulising part 74 of the nebuliser is controlled by a valve or manifold 75 which controls the pulses of pressurised air. The breathing pattern of a patient is detected by a sensor 76 which delivers information regarding the breathing pattern to the micro-controller 77 which, in turn, controls the manifold 75. Once a dose of medication has been delivered, indication means, such as a LED or buzzer 78 is activated by the micro-controller to indicate to the patient that treatment is complete.

A further embodiment of the invention is shown in Figure 10 which is a dosimetric spacer 80 including a holding chamber 81 having a port 82 towards one end thereof to which is connected a mouthpiece 83. An air pressure sensor 84 is located between the mouthpiece 83 and holding chamber 81. This sensor 84 measures the pressure within the mouthpiece, from which the rate of flow of air inspired and exhaled by the patient can be measured air. The mouthpiece 83 also includes a vent 85 which allows a patient to exhale through the mouthpiece 83 without filling the holding chamber 81. More is disclosed on the vent below.

Within the holding chamber, a piston 86 is disclosed to move longitudinally to vary the volume of air available in the holding chamber 81 to a patient during inspiration. The piston includes a toothed connecting rod 87 which extends through the end of the holding chamber 81 such that the teeth may be engaged by the finger of solenoid 88. An air inlet 89 is located at the left hand end of the holding chamber in order to allow air to enter or leave the space behind the piston as the piston moves to the right or left.

In use, the piston 86 is pulled back so as to fill the holding chamber 81 with air. The air within the holding chamber 81 is then loaded with medication, either in the form of liquid droplets, or in the form of a cloud of powder. This is delivered into the holding chamber 81 through the port 82, and normally requires the removal of the mouthpiece 83 to do so. A mouthpiece 83 then can be replaced, and a patient breathes in and out through the mouthpiece 83. During inspiration, a patient inspires the medication-laden air from the holding chamber 81, and during exhalation, the exhaled air is vented to atmosphere by the vent 85. During exhalation, the solenoid 88 locks the connecting rod 87 of the piston 86 so that it will not move and so that the holding chamber will not fill with exhaled air. However, in accordance with the invention, the piston 86 is only free to move during a proportion of the inhalation phase, and it will be locked stationary by the solenoid 88 during the inspiration by the patient of the end volume. Once the piston locks, the vent 85 is arranged such that the drop in pressure in the mouthpiece 83 caused by the locking of the piston 86 opens the vent 85 such that ambient air may be drawn into the mouthpiece. Of course, a separate vent might be included in the mouthpiece to carry out this function as appropriate.

The calculation of the pulse length during which the piston 86 is free to move to allow the dispensing of medication to the patient is determined in the same way as is described above in relation to the nebuliser. The inspiration of the patient during the previous three breaths is monitored by the sensor 84 such that the same calculations can be made as described above. On the subsequent breath, the sensor detects the commencement of a breath, and after the duration of the pulse, the piston is locked.

Such an arrangement reduces the wastage of medication which is present in the end volume of the air normally breathed in by the patient.

This invention is applicable to other types of medical inhalers. For example, as described in the introduction to this specification, a dry powder inhaler is disclosed in US 5694920 which uses a piezo-electric vibrator and an electrostatic charge plate to fluidise and disperse a dry powder into the air stream of the patient. The electrostatic charge plate can be operated in response to the patient's breathing pattern so as to produce pulses of the powder medication into the air stream leading to the patient. The length of the pulses can be determined in exactly the same way as in the embodiments previously described such that the dry powder is not dispersed into the end volume of the air stream leading to the patient.

**Claims**

1.  Drug delivery apparatus comprising:

    delivery means for delivering medication-laden air and air not carrying any medication to a patient for inspiration;
    monitoring means (9, 76) for monitoring a patient's breathing pattern; and
    control means (77) for controlling the said delivery means to selectively deliver the medication-laden air and air not carrying any medication,

    **characterised in that** the control means (77) is arranged to control the delivery means to deliver the medication-laden air in pulses, the length of which, and their proportion of the inspiratory phase of the breathing pattern being varied by the control means (77) depending on the breathing pattern monitored by the monitoring means (9,76).

2.  Drug delivery apparatus according to claim 1, wherein the control means (77) is arranged to control the length of the pulse on a breath-by-breath basis to adapt to changes in the monitored breathing pattern.

3.  Drug delivery apparatus according to claim I or 2, wherein the apparatus is a nebuliser (50,74) in which the means for selectively delivering medication-laden air is a means for atomising a medication, and the control means (77) controls the atomising means to atomise the medication in pulses.

4.  A nebuliser (50,74) according to claim 3, further comprising means for determining the duration of the pulse of atomisation, the determination means including means for measuring the tidal volume of a patient, timing means for measuring the duration of inspiration, means for storing an estimate of the volume of a patient's upper airway, and means for calculating the duration of the pulse on the basis of the tidal volume measured by the tidal volume measuring means, the duration of inspiration measured by the timing means, and the stored estimated volume of a patient's upper airway from the storage means.

5.  A nebuliser (50,74) according to claim 4, wherein the means for measuring the tidal volume of a patient includes means for measuring a patient's peak flow, and tidal volume prediction means for calculating the tidal volume on the basis of the peak flow from the peak flow measuring means, and the duration of inspiration measured by the timing means.

6.  A nebuliser according to claim 4 or 5, wherein some or all of the values used in the calculations are mean values derived from a number of earlier measurements of the breathing pattern of the patient.

7.  A nebuliser according to any one of claims 3 to 6, further comprising a source (70,51) of pressurised gas for atomising the medication, the source (70,51) including an accumulator for accumulating the gas under pressure, and means (73) for limiting the duration of the pulse so as to maintain the accumulator in a state where it is always under some pressure.

8.  A nebuliser according to claim 7, wherein the accumulator includes a valve (63) which, when the accumulator is full, vents gas to atmosphere.

9.  Drug delivery apparatus according to claim 1 or 2, wherein the apparatus is a spacer (80) in which the delivery means for selectively delivering medication-laden air is a holding chamber (81) for holding medication-laden air.

10. A spacer (80) according to claim 9, further comprising means for determining the duration of the pulse, the determination means including means for measuring the tidal volume of a patient, timing means for measuring the duration of inspiration, means for storing an estimate of the volume of a patient's upper airway, and means for calculating the duration of the pulse on the basis of the tidal volume measured by the tidal volume measuring means, the duration of inspiration measured by the timing means, and the stored estimated volume of a patient's upper airway from the storage means.

11. A spacer (80) according to claim 10, wherein the means for measuring the tidal volume of a patient includes means for measuring a patient's peak flow, and tidal volume prediction means for calculating the tidal volume on the basis of the peak flow from the peak flow measuring means, and the duration of inspiration measured by the timing means.

12. A spacer (80) according to claim 10 or 11, wherein some or all of the values used in the calculations are mean values derived from a number of earlier measurements of the breathing pattern of the patient.

13. A spacer (80) according to any one of claims 9 to 12, further comprising means (86) for reducing the volume of medication-laden air in the holding chamber (81) to permit the medication-laden air to be delivered to a patient.

14. A spacer (80) according to claim 13, wherein the means for reducing the volume of medication-laden air is a piston (86) moveable within the holding chamber (81).

15. A spacer (80) according to claim 13 or 14, further comprising means (87, 88) for controlling the movement of the means (86) for reducing the volume of the holding chamber (81) to deliver medication-laden air in pulses.

16. A spacer (80) according to any one of claims 9 to 15, further comprising a vent (85) which allows inspiration of ambient air not laden with medication outside of the time in which a pulse is present.

**Patentansprüche**

1. Abgabevorrichtung für Medikamente, mit:

   einer Abgabeeinrichtung zur Abgabe von mit Medikamenten beladener Luft, sowie von Luft, die keinerlei Medikamente trägt, zur Einatmung durch einen Patienten;
   einer Überwachungseinrichtung (9, 76) zur Überwachung eines Atmungsmusters des Patienten; und
   einer Steuereinrichtung (77) zur Steuerung der Abgabeeinrichtung zur selektiven Abgabe der mit Medikamenten beladenen Luft und der Luft, die keinerlei Medikamente trägt,

   **dadurch gekennzeichnet, daß** die Steuereinrichtung (77) dazu ausgebildet ist, die Abgabeeinrichtung so zu steuern, daß sie die mit Medikamenten beladene Luft in Impulsen abgibt, deren Länge und deren Verhältnis zur Einatmungsphase des Atmungsmusters durch die Steuereinrichtung (77) in Abhängigkeit von dem durch die Überwachungseinrichtung (9, 76) überwachten Atmungsmuster variiert wird.

2. Vorrichtung nach Anspruch 1, bei der die Steuereinrichtung (77) dazu ausgebildet ist, zur Anpassung an Änderungen in dem überwachten Atmungsmuster die Länge des Impulses Atemzug für Atemzug zu steuern.

3. Vorrichtung nach Anspruch 1 oder 2, bei der die Vorrichtung ein Zerstäuber (50, 74) ist, in dem die Einrichtung zur selektiven Abgabe der mit Medikamenten beladenen Luft eine Einrichtung zum Zerstäuben eines Medikaments ist und die Steuereinrichtung (77) die Zerstäubungseinrichtung so steuert, daß das Medikament impulsweise zerstäubt wird.

4. Zerstäuber (50, 74) nach Anspruch 3, mit einer Einrichtung zur Bestimmung der Dauer des Zerstäubungsimpulses, enthaltend eine Einrichtung zur Messung des Atemvolumens eines Patienten, einen Zeitgeber zur Messung der Dauer der Einatmung, eine Einrichtung zur Speicherung eines Schätzwertes für das Volumen des oberen Luftweges eines Patienten und eine Einrichtung zur Berechnung der Dauer des Impulses auf der Grundlage des von der Einrichtung zur Messung des Atemvolumens gemessenen Atemvolumens, der vom Zeitgeber gemessenen Dauer der Einatmung des in der Speichereinrichtung gespeicherten Schätzwertes für das Volumen des oberen Luftweges des Patienten.

5. Zerstäuber (50, 74) nach Anspruch 4, bei dem die Einrichtung zur Messung des Atemvolumens eines Patienten eine Einrichtung zur Messung eines Spitzenwertes des Atemstroms des Patienten und eine Atemvolumen-Vorhersageeinrichtung aufweist, zur Berechnung des Atemvolumens auf der Grundlage des Spitzenwertes des Atemstroms und der vom Zeitgeber gemessenen Dauer der Einatmung.

6. Zerstäuber nach Anspruch 4 oder 5, bei dem einige oder sämtliche der Werte, die in den Berechnungen verwendet werden, Mittelwerte sind, die aus einer Anzahl von früheren Messungen des Atmungsmusters des Patienten abgeleitet sind.

7. Zerstäuber nach einem der Ansprüche 3 bis 6, mit einer Quelle (71, 51) für Druckgas zum Zerstäuben des Medikaments, welche Quelle (70, 51) einen Akkumulator zum Sammeln des Gases unter Druck und eine Einrichtung (73) zur Begrenzung der Dauer des Impulses aufweist, um den Akkumulator in einem Zustand zu halten, in dem er stets unter gewissem Druck ist.

8. Zerstäuber nach Anspruch 7, bei dem der Akkumulator ein Ventil (63) aufweist, das, wenn der Akkumulator voll ist, Gas in die Atmosphäre abläßt.

9. Vorrichtung nach Anspruch 1 oder 2, bei der die Vorrichtung ein Dosimeter (80) ist, in dem die Abgabeeinrichtung zur selektiven Abgabe der mit Medikamenten beladenen Luft eine Haltekammer (81) zum Halten von mit Medikamenten beladener Luft ist.

10. Dosimeter (80) nach Anspruch 9, mit einer Einrichtung zur Bestimmung der Dauer des Zerstäubungsimpulses, enthaltend eine Einrichtung zur Messung des Atemvolumens eines Patienten, einen Zeitgeber zur Messung der Dauer der Einatmung, eine Einrichtung zur Speicherung eines Schätzwertes für das Volumen des oberen Luftweges eines Patienten und eine Einrichtung zur Berechnung der Dauer des Impulses auf der Grundlage des von der Einrichtung zur Messung des Atemvolumens gemessenen Atemvolumens, der vom Zeitgeber gemessenen Dauer der Einatmung des in der Speichereinrichtung gespeicherten Schätzwertes für das Volumen des oberen Luftweges des Patienten.

11. Dosimeter (80) nach Anspruch 10, bei dem die Einrichtung zur Messung des Atemvolumens eines Patienten eine Einrichtung zur Messung eines Spitzenwertes des Atemstroms des Patienten und eine Atemvolumen-Vorhersageeinrichtung aufweist, zur Berechnung des Atemvolumens auf der Grundlage des Spitzenwertes des Atemstroms und der vom Zeitgeber gemessenen Dauer der Einatmung.

12. Dosimeter (80) nach Anspruch 10 oder 11, bei dem einige oder sämtliche der Werte, die in den Berechnungen verwendet werden, Mittelwerte sind, die aus einer Anzahl von früheren Messungen des Atmungsmusters des Patienten abgeleitet sind.

13. Dosimeter (80) nach einem der Ansprüche 9 bis 12, mit einer Einrichtung (86) zur Verringerung des Volumens der mit Medikamenten beladenen Luft in der Haltekammer (81), um die Abgabe der mit Medikamenten beladenen Luft an einen Patienten zu ermöglichen.

14. Dosimeter (80) nach Anspruch 13, bei dem die Einrichtung zur Verringerung des Volumens der mit Medikamenten beladenen Luft ein Kolben (86) ist, der in der Haltekammer (81) beweglich ist.

15. Dosimeter (80) nach Anspruch 13 oder 14, mit einer Einrichtung (87, 88) zur Steuerung der Bewegung der Einrichtung (86) zur Verringerung des Volumens der Haltekammer (81) zur Abgabe der mit Medikamenten beladenen Luft in Impulsen.

16. Dosimeter (80) nach einem der Ansprüche 9 bis 15, mit einer Belüftungsöffnung (85), die das Einatmen von nicht mit Medikamenten beladener Luft außerhalb der Zeit erlaubt, in der ein Impuls vorhanden ist.

**Revendications**

1. Appareil d'administration de médicament comprenant :

   des moyens d'administration pour fournir de l'air chargé en médicament et de l'air exempt de tout médicament à un patient pour inspiration ;
   des moyens de contrôle (9, 76) pour contrôler le rythme respiratoire d'un patient ; et
   des moyens de commande (77) pour commander lesdits moyens d'administration pour fournir de manière sélective l'air chargé en médicament et l'air exempt de tout médicament,

   **caractérisé en ce que** les moyens de commande (77) sont prévus pour commander les moyens d'administration pour fournir l'air chargé en médicament par impulsions, dont la longueur, et la proportion dans la phase inspiratoire du diagramme de respiration sont ajustables à l'aide des moyens de commande (77) en fonction du rythme respiratoire contrôlé par les moyens de contrôle (9, 76).

2. Appareil d'administration de médicament selon la revendication 1, dans lequel les moyens de commande (77) sont prévus pour commander la longueur de l'impulsion sur une base cycle par cycle de respiration afin de s'adapter aux changements dans le rythme respiratoire contrôlé.

3. Appareil d'administration de médicament selon la revendication 1 ou 2, dans lequel l'appareil est un nébuliseur (50, 74) dans lequel les moyens pour distribuer de manière sélective de l'air chargé en médicament sont des moyens pour atomiser un médicament, et les moyens de commande (77) commandent les moyens d'atomisation pour atomiser le médicament par impulsions.

4. Nébuliseur (50, 74) selon la revendication 3, comprenant en outre des moyens pour déterminer la durée de l'impulsion d'atomisation, lesdits moyens de détermination comprenant des moyens pour mesurer le volume respiratoire courant d'un patient, des moyens de mesure de temps pour mesurer la durée d'inspiration, des moyens pour mémoriser une estimation du volume de la voie respiratoire supérieure d'un patient, et des moyens pour calculer la durée de l'impulsion sur la base du volume respiratoire courant mesuré par les moyens de mesure de volume respiratoire courant, de la durée d'inspiration mesurée par les moyens de mesure de temps, et du volume estimé du volume de la voie respiratoire supérieure d'un patient mémorisé par les moyens de mémorisation.

5. Nébuliseur (50, 74) selon la revendication 4, dans lequel les moyens pour mesurer le volume respiratoire courant d'un patient comprennent des moyens pour mesurer le débit de pointe d'un patient, et des moyens de prédiction du volume respiratoire courant pour calculer le volume respiratoire courant sur la base du débit de pointe obtenu à partir des moyens de mesure de débit de pointe, et de la durée d'inspiration mesurée par les moyens de mesure de temps.

6. Nébuliseur selon la revendication 4 ou 5, dans lequel tout ou partie des valeurs utilisées dans les calculs sont des valeurs moyennes dérivées d'une série de mesures antérieures du cycle respiratoire du patient.

7. Nébuliseur selon l'une quelconque des revendications 3 à 6, comprenant en outre une source (70, 51) de gaz sous pression pour atomiser le médicament, la source (70, 51) comprenant un accumulateur pour stocker le gaz sous pression, et des moyens (73) pour limiter la durée de l'impulsion de sorte à maintenir l'accumulateur dans un état où il est toujours sous une certaine pression.

8. Nébuliseur selon la revendication 7, dans lequel l'accumulateur comprend une soupape (63) qui, lorsque l'accumulateur est ouvert, laisse s'échapper le gaz dans l'atmosphère.

9. Appareil d'administration de médicament selon la revendication 1 ou 2, dans lequel l'appareil est un dispositif d'espacement (80) dans lequel les moyens d'administration pour fournir de manière sélective de l'air chargé en médicament est une chambre de retenue (81) pour stocker de l'air chargé en médicament.

10. Dispositif d'espacement (80) selon la revendication 9, comprenant en outre des moyens pour déterminer la durée de l'impulsion, lesdits moyens de détermination comprenant des moyens pour mesurer le volume respiratoire courant d'un patient, des moyens de mesure de temps pour mesurer la durée d'inspiration, des moyens pour mémoriser une estimation du volume de la voie respiratoire supérieure d'un patient, et des moyens pour calculer la durée de l'impulsion sur la base du volume respiratoire courant mesuré par les moyens de mesure de volume respiratoire courant, de la durée d'inspiration mesurée par les moyens de mesure de temps, et du volume estimé du volume de la voie respiratoire supérieure d'un patient mémorisé par les moyens de mémorisation.

11. Dispositif d'espacement (80) selon la revendication 10, dans lequel les moyens pour mesurer le volume respiratoire courant d'un patient comprend des moyens pour mesurer le débit de pointe d'un patient, et des moyens de prédiction du volume respiratoire courant pour calculer le volume respiratoire courant sur la base du débit de pointe obtenu à partir des moyens de mesure de débit de pointe, et de la durée d'inspiration mesurée par les moyens de mesure de temps.

12. Dispositif d'espacement (80) selon la revendication 10 ou 11, dans lequel tout ou partie des valeurs utilisées dans les calculs sont des valeurs moyennes dérivées d'une série de mesures antérieures du cycle respiratoire du patient.

13. Dispositif d'espacement (80) selon l'une quelconque des revendications 9 à 12, comprenant en outre des moyens (86) pour réduire le volume d'air chargé en médicament dans la chambre de retenue (81) pour permettre d'administrer à un patient l'air chargé en médicament.

**14.** Dispositif d'espacement (80) selon la revendication 13, dans lequel les moyens pour réduire le volume d'air chargé en médicament sont constitués par un piston (86) déplaçable à l'intérieur de la chambre de retenue (81).

**15.** Dispositif d'espacement (80) selon la revendication 13 ou 14, comprenant en outre des moyens (87, 88) pour commander le déplacement des moyens (86) pour réduire le volume de la chambre de retenue (81) pour fournir de l'air chargé en médicament par impulsions.

**16.** Dispositif d'espacement (80) selon l'une quelconque des revendications 9 à 15, comprenant en outre un évent (85) qui permet d'inspirer de l'air ambiant non chargé en médicament en dehors des périodes pendant lesquelles une impulsion a lieu.

Pulse time = 50% sum $\dfrac{(T1 + T2 + T3)}{3}$

Dose = Sum (P1 + P2 + ....)

Fig.1.

Fig.10.

Fig.2.

Fig.3.

Fig.4.

Fig.5.

Fig.6.

Fig.7.

Fig.8.

Fig.9.